# EUROPÄISCHE PATENTSCHRIFT

(11) **EP 3 952 798 B1**
(45) Veröffentlichungstag und Bekanntmachung des Hinweises auf die Patenterteilung: **01.04.2026**
(21) Anmeldenummer: 20724480.7
(22) Anmeldetag: 06.05.2020
(51) Int. Cl.: A61F 2/915

(54) **STENT**
STENT
STENT

(30) Priorität: 16.05.2019 DE 102019112971
(43) Veröffentlichungstag der Anmeldung: 16.02.2022
(73) Patentinhaber: optimed medizinische Instrumente GmbH, 76275 Ettlingen (DE)
(72) Erfinder: NENNIG, Ernst, 76131 Karlsruhe (DE); FISCHER, Harald, 76356 Weingarten (DE)
(74) Vertreter: Manitz Finsterwald Patent- und Rechtsanwaltspartnerschaft mbB
(86) Internationale Anmeldenummer: PCT/EP2020/062554
(87) Internationale Veröffentlichungsnummer: WO 2020/229255

(56) Entgegenhaltungen:
- EP-B1- 3 250 247
- WO-A1-2012/159454
- WO-A1-2017/200956
- DE-A1- 102015 101 264
- US-A1- 2008 275 537
- US-A1- 2011 004 291
- US-A1- 2011 230 957
- US-A1- 2012 277 844
- US-A1- 2014 081 377
- US-A1- 2018 104 044

## Beschreibung

Die vorliegende Erfindung betrifft einen Stent zur transluminalen Implantation in Hohlorgane, insbesondere in Blutgefäße, Harnleiter, Speiseröhren, Kolon, Duodenum oder Gallenwege, mit einem im Wesentlichen röhrenförmigen Körper, der sich entlang einer axialen Richtung erstreckt und von einem komprimierten Zustand mit einem ersten Querschnittsdurchmesser in einen expandierten Zustand mit einem vergrößerten zweiten Querschnittsdurchmesser überführbar ist. Der Stent umfasst eine Vielzahl von Zellen, welche von durch den röhrenförmigen Körper gebildeten stegartigen Umrandungselementen definiert werden. Die Umrandungselemente umfassen zumindest eine ringförmig um die axiale Richtung umlaufende Stützstrebe.

Stents dieser Art werden zur Rekanalisation von krankhaft veränderten Hohlorganen eingesetzt. Dabei werden die Stents im komprimierten Zustand mit dem ersten Querschnittsdurchmesser über einen Einführkatheter an die zu behandelnde Stelle innerhalb des Hohlorgans gebracht, wo sie durch unterschiedliche Maßnahmen auf einen Durchmesser, der dem Durchmesser des gesunden Hohlorgans entspricht, expandiert werden, so dass eine Stützwirkung des Hohlorgans, beispielsweise einer Gefäßwand, erreicht wird. Nach dem Expandieren weist der Stent insbesondere den zweiten Querschnittsdurchmesser auf.

Ein Stent gemäß dem Oberbegriff des Anspruchs 1 ist aus der US 2018/104044 A1 bekannt. Des Weiteren beschreibt die US 2011/004291 A1 einen Stent gemäß dem Oberbegriff des Anspruchs 11.

Zur Bereitstellung der Stützwirkung ist es notwendig, dass der Stent eine möglichst hohe Aufstellkraft generieren kann, d.h. eine Kraft in radialer Richtung, die beispielsweise gegen die Wand eines Blutgefäßes drückt.

Es ist daher die der Erfindung zugrundeliegende Aufgabe, einen Stent der eingangs genannten Art derart weiterzubilden, dass eine möglichst hohe Aufstellkraft bereitgestellt wird, insbesondere auch dann, wenn der Stent aus relativ weichen Materialien geformt ist.

Diese Aufgabe wird erfindungsgemäß durch einen Stent mit den Merkmalen des Anspruchs 1 und durch einen Stent mit den Merkmalen des Anspruchs 11 gelöst.

Der erfindungsgemäße Stent zeichnet sich dadurch aus, dass die Stützstrebe zumindest einen V-förmigen Stützabschnitt aufweist, welcher einen Stegwinkel von 90° bis 150° umfasst, wenn der Stent den zweiten Querschnittsdurchmesser aufweist. Die V-Form des Stützabschnitts kann durch zwei Schenkel gebildet sein. Die Schenkel können Teil der Umrandungselemente sein.

Die Erfindung setzt auf der Erkenntnis auf, dass durch den relativ großen Stegwinkel von 90° bis 150° eine hohe radiale Aufstellkraft erzeugt werden kann, auch wenn der Stent beispielsweise aus Materialien mit eher geringerer Zugfestigkeit geformt ist. Im Gegensatz zu beispielsweise üblichen Koronarstents, welche Stegwinkel im Bereich zwischen 60° und 80° aufweisen, kann erfindungsgemäß eine Reihe von Vorteilen erzielt werden, wie nachfolgend noch detailliert erläutert wird.

Unter dem Stegwinkel ist dabei insbesondere der Winkel zu verstehen, der von den beiden Schenkeln des V-förmigen Stützabschnitts aufgespannt bzw. definiert wird. Der Stegwinkel wird dabei, sofern hierin nicht anders angegeben, immer bestimmt, wenn sich der Stent im expandierten Zustand mit dem zweiten Querschnittsdurchmesser befindet, wobei der zweite Querschnittsdurchmesser üblicherweise den Nenndurchmesser definiert. Der Nenndurchmesser ist der Durchmesser, der beim regulären Einsatz des Stents dauerhaft benötigt wird.

Generell kann der Stent aus den genannten Umrandungselementen geformt sein, wobei die Umrandungselemente auch als Stentstreben bezeichnet werden können, welche gemeinsam ein Gittergerüst bilden, welches wiederum den röhrenförmigen Körper des Stents definiert.

Die Umrandungselemente bilden dabei bevorzugt eine Vielzahl von Zellen, wobei eine jeweilige Zelle das jeweilige, sie begrenzende, Umrandungselement umfasst und mittels weiterer Umrandungselemente mit weiteren Zellen verbunden sein kann.

Bevorzugte Ausführungsformen der Erfindung sind der Beschreibung, den Unteransprüchen und den Zeichnungen zu entnehmen.

Gemäß einer ersten vorteilhaften Ausführungsform weist der Stegwinkel einen Winkel von 90° bis 140°, bevorzugt von 100° bis 130°, auf. Weiter bevorzugt kann der Stegwinkel einen Winkel von 105° bis 115° aufweisen. Die hierin genannten Winkelangaben sind derart zu verstehen, dass die genannten Grenzen jeweils eingeschlossen sind. Insbesondere kann der Stegwinkel 110° betragen. Es hat sich herausgestellt, dass in dem Bereich um 110° eine besonders hohe radiale Aufstellkraft erzielbar ist.

Nur zur Klarstellung sei bemerkt, dass die vorstehend genannten Stegwinkel sich wieder auf den Zustand beziehen, wenn der Stent auf den zweiten Querschnittsdurchmesser aufgeweitet ist. Im ersten Querschnittsdurchmesser, in welchem der Stent verkleinert in den Körper einführbar ist, kann der Winkel des V-förmigen Stützabschnitts deutlich geringer sein und insbesondere sogar 0° betragen, wenn die Schenkel parallel verlaufen. Genauer gesagt, kann der Winkel des V-förmigen Stützabschnitts im komprimierten Zustand mit dem ersten Querschnittsdurchmesser insbesondere kleiner als 30°, bevorzugt kleiner als 15°, sein.

Erfindungsgemäß umfassen die Umrandungselemente zumindest bereichsweise ein bioresorbierbares Material, welches aus Zink (Zn) besteht oder Zink enthält. Durch die Verwendung eines bioresorbierbaren Materials verbleibt der Stent nicht für unbegrenzte Zeitdauer im Körper oder muss operativ entfernt werden. Stattdessen löst sich das Material nach einigen Monaten im Körper auf und wird über natürliche Stoffwechselvorgänge absorbiert und vollständig ausgeschieden. Auf diese Weise können Biokompatibilitätsprobleme und körpereigene Abwehrreaktionen minimiert werden, welche zu Wiederverschlüssen von Gefäßen (beispielsweise durch späte Arteriosklerose oder Thrombenbildung) führen können.

Als bioresorbierbare Materialien sind insbesondere polymere Werkstoffe bekannt, beispielsweise Poly-Milchsäure (PLA) oder Poly-L-Milchsäure (PLLA). Solche polymeren Materialien weisen allerdings eine geringe mechanische Stabilität auf, so dass bevorzugt ein bioresorbierbares Material mit einem Zink-Anteil eingesetzt wird. Durch die Verwendung von Zink oder einer Zink-Legierung kann auch die Röntgensichtbarkeit des Stents im Vergleich zu PLA, PLLA oder Magnesiumlegierungen deutlich gesteigert werden. Hierdurch wird das Einsetzen des Stents unter Röntgenbeobachtung deutlich erleichtert und es sind nicht zwangsläufig separate Röntgenmarker notwendig.

Andere bekannte bioresorbierbare Werkstoffe sind Magnesium und magnesiumhaltige Legierungen, wie vorstehend bereits angedeutet. Im Körper reagiert Magnesium nachteiligerweise mit dem im Körpergewebe enthaltenen Wasser unter Energiefreisetzung zu Magnesiumhydroxid und Wasserstoff. Der Wasserstoff liegt dann gasförmig vor und kann bei entsprechender Konzentration beispielsweise im Blutkreislauf zu lebensbedrohlichen Embolien führen. Stents aus Magnesiumlegierungen sind daher üblicherweise aufwändig mit PLLA-Beschichtungen versehen, um den Abbauprozess kontrollieren zu können. Außerdem sind die mechanischen Eigenschaften vor allem in Bezug auf die Bruchdehnung schlechter als die von Zinklegierungen.

Insbesondere kann als bioresorbierbares Material reines Zink oder eine Zink-Legierung eingesetzt werden. Die Zink-Legierung kann beispielsweise durch Beimischung von Silber (Ag) und/oder Titan (Ti) erzeugt werden. Bevorzugt kann die Zink-Legierung 90,0 bis 99,95 Massen-% Zink und 0,05 bis 10,0 Massen-% Silber aufweisen. Ebenfalls können 0,05 bis 10,0 Massen-% Titan der Zink-Legierung beigemischt werden. Bevorzugt wird allerdings ein Anteil von 0,9 bis 4,0 Massen-% Silber und/oder Titan verwendet, wobei die Zink-Legierung im Übrigen aus Zink besteht. Besonders bevorzugt werden neben Zink nur 3,0 Massen-% Silber und/oder Titan verwendet, insbesondere zwischen 2,8 und 3,2 Massen-%. Eine Kombination von 3,0 Massen-% Silber und 97 Massen-% Zink hat sich für die hierin beschriebenen Stents als vorteilhaft bezüglich der mechanischen Stabilität herausgestellt.

Gemäß einer weiteren vorteilhaften Ausführungsform besteht das bioresorbierbare Material aus Zink und Silber, wobei das bioresorbierbare Material 90,0 bis 99,95 Massen-% Zink und 0,05 bis 10,0 Massen-% Silber enthält. Das bioresorbierbare Material kann dementsprechend ausschließlich aus Zink und Silber bestehen. Eine solche Legierung ist in der europäischen Patentanmeldung mit der Anmeldenummer EP 16 702 899.2 beschrieben.

Die hierin genannten Zink-Legierungen aus Zink und Silber und/oder Titan können beispielsweise eine Zugfestigkeit von 180 bis 210 MPa, bevorzugt von 190 bis 200 MPa aufweisen. Die Zugfestigkeit liegt dabei niedriger, als beispielsweise bei einem Edelstahl 316L, welcher nicht bioresorbierbar ist und eine Zugfestigkeit von etwa 586 MPa aufweist. Durch den erfindungsgemäß gewählten Stegwinkel kann allerdings trotzdem ein Stent mit hoher radialer Aufstellkraft geschaffen werden.

Die hierin genannten Zink-Legierungen können zudem eine Bruchdehnung im Bereich von 80 bis 180%, beispielsweise von 80 bis 100%, bevorzugt im Bereich von 90 bis 100% aufweisen. Die Bruchdehnung gibt an, um wieviel Prozent ein Material gedehnt werden kann, bis es bricht.

Dieser hohe Wert der Bruchdehnung für die genannten Zink-Legierungen ermöglicht deutlich flexiblere Stützstreben des Stents, als dies beispielsweise mit dem Edelstahl 316L (Bruchdehnung 35%) oder mit reinem Zink (Bruchdehnung 8%) möglich wäre. Hierdurch wird es insbesondere ermöglicht, den großen erfindungsgemäßen Stegwinkel zu realisieren und insbesondere auch den großen Winkelunterschied des V-förmigen Stützabschnitts zwischen dem Zustand mit komprimiertem ersten Querschnittsdurchmesser und expandiertem zweiten Querschnittsdurchmesser zu erzielen.

Es ist zu beachten, dass die hierin angegebenen Werte für die Bruchdehnung für das tatsächlich verarbeitete bioresorbierbare Material des Stents gelten. Vor der tatsächlichen Verarbeitung kann das bioresorbierbare Material beispielsweise durch Strangpressen und/oder Rohrziehen bearbeitet werden, wodurch sich die Bruchdehnung (und auch andere Materialeigenschaften) im Vergleich zur reinen Legierung noch signifikant verändern kann/können.

Gemäß einer weiteren vorteilhaften Ausführungsform umfasst die Stützstrebe mehrere V-förmige Stützabschnitte, welche jeweils Stegwinkel im vorgenannten Bereich aufweisen, so dass sich zumindest bereichsweise eine Zickzack-Form der Stützstrebe ergibt. Es können also mehrere V-förmige Stützabschnitte derart hintereinander angeordnet sein, dass sich eine Zickzackform der Stützstrebe ergibt.

Die durch die Schenkel definierten "Öffnungen" der V-förmigen Stützabschnitte weisen dabei jeweils abwechselnd in entgegengesetzte Richtungen.

Die Stegwinkel der einzelnen Stützabschnitte können jeweils unterschiedlich sein, aber in einem der vorgenannten Bereiche liegen. Alternativ können die Stegwinkel zumindest zum Teil, oder alle, gleich sein, wobei hierbei eine Abweichung bis 10%, bevorzugt bis 5%, besonders bevorzugt bis 3%, als gleich angesehen wird. Durch gleiche oder zumindest ähnliche Stegwinkel kann ein homogener Aufbau des Stents erreicht werden. Insbesondere kann die Stützstrebe aus beispielsweise 8, 12 oder 16 V-förmigen Stützabschnitten geformt sein.

Erfindungsgemäß ist im Bereich des Stegwinkels ein Längsverbinder an der Stützstrebe angebracht, welcher die Stützstrebe mit zumindest einer weiteren Stützstrebe verbindet. Der Längsverbinder kann insbesondere an dem Punkt angebracht sein, an welchem die beiden Schenkel des V-förmigen Stützabschnitts aufeinandertreffen. Der Stegwinkel wird bevorzugt ohne Berücksichtigung des Längsverbinders ermittelt.

Bevorzugt erstreckt sich der Längsverbinder zumindest im Wesentlichen parallel zu der axialen Richtung und damit bevorzugt etwa im rechten Winkel zu der Stützstrebe. Der Längsverbinder ist insbesondere ein Umrandungselement, wobei zwei Längsverbinder und die zugehörigen Abschnitte der Stützstreben eine Zelle aufspannen bzw. definieren.

Der Längsverbinder kann bevorzugt einen, in Draufsicht, etwa kreisförmigen oder elliptischen Positionsmarker aufweisen. Der Positionsmarker kann eine Verdickung des bioresorbierbaren Materials umfassen oder aus einer solchen bestehen. Der Positionsmarker kann insbesondere eine Positionierung des Stents auf einem Katether ermöglichen. Der Positionsmarker ist bevorzugt angrenzend an oder benachbart zu der Stützstrebe angeordnet.

Entlang des Verlaufs der Stützstrebe um die axiale Richtung kann der Stegwinkel jeweils abwechselnd auf einer dem Längsverbinder abgewandten Seite und auf einer dem Längsverbinder zugewandten Seite der Stützstrebe liegen. Liegt der Stegwinkel auf der dem Längsverbinder zugewandten Seite, so kann der Längsverbinder etwa mittig in dem Stegwinkel liegen.

Zudem kann ein Längsverbinder nur im Bereich jedes zweiten Stegwinkels angebracht sein. Entlang der Umfangsrichtung einer Stützstrebe gesehen, können die Längsverbinder abwechselnd auf gegenüberliegenden Seiten der Stützstrebe angebracht sein. Anders ausgedrückt, können mehr V-förmige Stützabschnitte pro Stützstrebe vorhanden sein, als Längsverbinder. Alternativ können genau gleich viele V-förmige Stützabschnitte vorhanden sein, wie Längsverbinder.

Bevorzugt können an jeder Stützstrebe mehrere Längsverbinder, insbesondere genau zwei oder drei Längsverbinder, auf derselben Seite angebracht sein. Dies bedeutet, zwei benachbarte Stützstreben werden durch genau zwei oder drei Längsverbinder miteinander verbunden. Durch die Verbindung mit zwei oder drei Längsverbindern ist es möglich, den Stent flexibel auszubilden, so dass sich dieser gut an Krümmungen und Kurven des zu stützenden Hohlorgans anpassen kann. Zugleich wird durch die Stützstreben eine hohe radiale Aufstellkraft bereitgestellt.

Der Stent kann insbesondere einen Mittelteil aufweisen, welcher ausschließlich aus Stützstreben und den die Stützstreben verbindenden Längsverbindern besteht. Der Mittelteil befindet sich in der axialen Richtung gesehen, zwischen den beiden Enden des Stents.

Gemäß einer weiteren vorteilhaften Ausführungsform läuft die Stützstrebe zumindest im Wesentlichen derart radial um die axiale Richtung um, dass die axiale Richtung einen Normalenvektor auf die von der Stützstrebe definierte Ebene bildet. Für die Ermittlung der von der Stützstrebe definierten Ebene wird eine Zick-zack-Form der Stützstrebe nicht berücksichtigt, sondern gegebenenfalls herausgemittelt. Anders ausgedrückt, ist die Stützstrebe nicht seitlich, schräg oder zu der axialen Richtung verkantet angeordnet. Stattdessen steht die axiale Richtung auf der von der Stützstrebe definierten Ebene senkrecht. Die Stützstreben überkreuzen sich folglich insbesondere auch nicht. Bevorzugt sind sämtliche Stützstreben auf diese Weise angeordnet.

Gemäß einer weiteren vorteilhaften Ausführungsform ist der Stent auf einen dritten Querschnittsdurchmesser aufweitbar, welcher größer als der zweite Querschnittsdurchmesser ist, wobei der Stegwinkel sich auf zumindest 120°, bevorzugt auf zumindest 140° oder 160°, vergrößert, wenn der Stent den dritten Querschnittsdurchmesser aufweist. Insbesondere kann beim dritten Querschnittsdurchmesser auch ein Stegwinkel von zumindest 165° oder 170° vorliegen. Der Stegwinkel kann beim dritten Querschnittsdurchmesser auch um zumindest 10° oder 20° größer sein als beim zweiten Querschnittsdurchmesser.

Beispielsweise im Koronarbereich kann es notwendig sein, beim Einsetzen des Stents den Stent kurzfristig auf den dritten Querschnittsdurchmesser zu vergrößern, wodurch der Stent dann besser in das jeweilige Blutgefäß eingebettet wird und ein ausreichend dimensioniertes Lumen freihält, um einen ungehinderten Blutfluss zu gewährleisten. Hierdurch wird die Anhaftung von Blutplättchen unterdrückt, die sonst zu einer Thrombose führen könnten.

Der dritte Querschnittsdurchmesser kann zumindest 7 bis 20% (z.B. 10% oder 14%) größer sein als der zweite Querschnittsdurchmesser. Insbesondere aufgrund der hierin erwähnten Zink-Legierung kann das Material des Stents derart flexibel sein, dass sich auch die erwähnten Stegwinkel beim dritten Querschnittsdurchmesser erreichen lassen, ohne dass ein Materialbruch erfolgt. Der maximale Stegwinkel beim dritten Querschnittsdurchmesser kann bei 170°, bevorzugt bei maximal 150° liegen.

Gemäß einer weiteren vorteilhaften Ausführungsform ist der Stent, bis auf die Endbereiche in der axialen Richtung, gleichartig aufgebaut. Dies bedeutet, der Stent ist überall gleich aufgebaut und kann beispielsweise nur an seinen Enden von diesem gleichartigen Aufbau abweichen. Unter den Enden in der axialen Rich-tung werden die Enden verstanden, an welchen beispielsweise ein Blutein- oder -austritt erfolgt, sofern der Stent in einer abzweigungsfreien Blutbahn eingesetzt wird.

Alternativ ist es auch möglich, dass innerhalb eines vordefinierten Gefäßabzweigungs-Flächenbereichs die Umrandungselemente eine geringere Masse aufweisen als die Umrandungselemente in einem anderen Flächenbereich gleicher Größe, wobei die geringere Masse beispielsweise durch dünnere Umrandungsele-mente erzielt wird. Der vordefinierte Gefäßabzweigungs-Flächenbereich ist eine insbesondere gekrümmte Fläche, die sich ergibt, wenn sich der Stent im expandierten Zustand mit dem zweiten Querschnittsdurchmesser befindet.

Der Gefäßabzweigungs-Flächenbereich dient dazu, an einer Gefäßabzweigung positioniert zu werden, wobei dann durch die geringere Masse im Bereich des Gefäßabzweigungs-Flächenbereichs ein Dilatieren des Abzweigs leichter möglich ist. Hierdurch kann also eine Aussparung im Bereich des Gefäßabzweigungs-Flächenbereichs geschaffen werden, die z.B. einen ungehinderten Blutfluss bei der Gefäßabzweigung ermöglicht.

Um die geringere Masse zu erzielen, kann die Masse pro Weglänge der Umrandungselemente beispielsweise um zumindest 15%, bevorzugt um zumindest 25% kleiner sein als bei den übrigen Umrandungselementen. Dementsprechend können die Umrandungselemente und damit z.B. auch ein Bereich einer Stützstrebe, welcher durch den Gefäßabzweigungs-Flächenbereich verläuft, um z.B. 25% dünner oder schmaler sein.

Gemäß einer weiteren vorteilhaften Ausführungsform sind innerhalb des Gefäßabzweigungs-Flächenbereichs keine Längsverbinder angeordnet. Anders ausgedrückt, sind innerhalb des Gefäßabzweigungs-Flächenbereichs ausschließlich Stützstreben, z.B. mit ihren V-förmigen Stützabschnitten, vorgesehen. Durch das Weglassen der Längsverbinder ist die Masse der Umrandungselemente ebenfalls geringer. Ein Dilatieren des Abzweigs wird auf diese Weise noch weiter vereinfacht, gleichwohl bleibt die radiale Festigkeit aber erhalten.

Gemäß einer weiteren vorteilhaften Ausführungsform sind innerhalb des Gefäßabzweigungs-Flächenbereichs und/oder angrenzend an den Gefäßabzweigungs-Flächenbereich ein oder mehrere Röntgenmarker angeordnet. Die Röntgenmarker können ein röntgendichtes Material aufweisen, beispielsweise Tantal. Das röntgendichte Material kann in einer ösenförmigen Struktur der Umrandungselemente gehalten werden. Insbesondere können z.B. vier Röntgenmarker vorgesehen sein, die jeweils in gleichmäßigen Abständen an der Grenze des Gefäßabzweigungs-Flächenbereichs angeordnet sind. Durch die derart angeordneten Röntgenmarker wird die korrekte Positionierung des Stents bzw. des Gefäßabzweigungs-Flächenbereichs an einer Gefäßabzweigung unter Röntgenbeobachtung deutlich erleichtert.

Gemäß einer weiteren vorteilhaften Ausführungsform sind die Umrandungselemente zumindest bereichsweise mit einem Medikament versehen, wobei das Medikament vom Stent bevorzugt über einen vorbestimmten Zeitraum abgegeben wird. Das Medikament kann anti-proliferativ wirken, um ein Zuwuchern des Stents mit Gewebe zu vermeiden. Beispielsweise können als Medikament Antiproliferative der Limus-Gruppe, der Statine, der P2Y12-Antagonisten oder der Thrombinantagonisten verwendet werden.

Gemäß einer weiteren vorteilhaften Ausführungsform weisen die Umrandungselemente eine Wandstärke von maximal 4%, bevorzugt von maximal 2%, weiter bevorzugt von maximal 1,5%, des zweiten Querschnittsdurchmessers auf. Die Breite der Umrandungselemente, d.h. die Dimension in der Umfangsrichtung gesehen, kann bevorzugt maximal 4%, weiter bevorzugt maximal 2%, besonders bevorzugt maximal 1,7%, des zweiten Querschnittsdurchmessers aufweisen. Die Umrandungselemente können beispielsweise eine Breite von etwa 105 bis 120 µm und eine Wandstärke von 90 bis 115 µm aufweisen.

Der Stent kann damit Umrandungselemente umfassen, welche etwa 25 bis 40% schmaler und/oder dünner sind als Umrandungselemente herkömmlicher bioresorbierbarer Stents aus PLLA. Durch die besonders dünnen und schmalen Umrandungselemente wird ein Thromboserisiko weiter verhindert, da das Anlagern von Blutplättchen erschwert wird.

Insbesondere kann es sich bei dem Stent um einen Koronarstent handeln, dessen zweiter Querschnittsdurchmesser beispielsweise maximal 2 mm oder 4 mm beträgt.

Weiterer Gegenstand der Erfindung ist ein Stent zur transluminalen Implantation in Hohlorgane, insbesondere in Blutgefäße, Harnleiter, Speiseröhren, Colon, Duodenum oder Gallenwege, mit einem im Wesentlichen röhrenförmigen Körper, der sich entlang einer axialen Richtung erstreckt und von einem komprimierten Zustand mit einem ersten Querschnittsdurchmesser in einen expandierten Zustand mit einem vergrößerten zweiten Querschnittsdurchmesser überführbar ist. Der Stent umfasst eine Vielzahl von Zellen, welche von durch den röhrenförmigen Körper gebildeten stegartigen Umrandungselementen definiert werden. Die Umrandungselemente umfassen zumindest eine ringförmig um die axiale Richtung umlaufende Stützstrebe, wobei ein Längsverbinder an der Stützstrebe angebracht ist, welcher die Stützstrebe mit zumindest einer weiteren Stützstrebe verbindet. Der Stent zeichnet sich dadurch aus, dass innerhalb eines vordefinierten Gefäßabzweigungs-Flächenbereichs keine Längsverbinder angeordnet sind und die Umrandungselemente zumindest bereichsweise ein bioresorbierbares Material umfassen, welches Zink umfasst.

Durch das Weglassen der Längsverbinder ist die Masse der Umrandungselemente geringer und ein Dilatieren des Abzweigs wird vereinfacht, wie oben bereits ausgeführt. Anders ausgedrückt, sind innerhalb des Gefäßabzweigungs-Flächenbereichs ausschließlich Stützstreben, z.B. mit V-förmigen Stützabschnitten, vorgesehen.

Der Gefäßabzweigungs-Flächenbereichs kann insbesondere die Größe einer Arterie oder Vene aufweisen, welche in ein von dem Stent abgestütztes Blutgefäß im Bereich des Stents einmündet.

Gemäß einer Ausführungsform sind innerhalb des Gefäßabzweigungs-Flächenbereichs und/oder angrenzend an den Gefäßabzweigungs-Flächenbereich ein oder mehrere Röntgenmarker oder Positionsmarker angeordnet.

Gemäß einer Ausführungsform weisen die Umrandungselemente innerhalb des vordefinierten Gefäßabzweigungs-Flächenbereichs eine geringere Masse auf, als die Umrandungselemente in einem anderen Flächenbereich gleicher Größe, wobei die geringere Masse beispielsweise durch dünnere Umrandungselemente erzielt wird.

Zu dem weiteren offenbarten Stent gelten die hierin getroffenen Aussagen zu dem zuerst genannten Stent entsprechend, dies gilt insbesondere für Vorteile und bevorzugte Ausführungsformen.

Nachfolgend wird die Erfindung rein beispielhaft unter Bezugnahme auf die Zeich-nungen beschrieben. Es zeigen:
- Fig. 1: eine Außenansicht eines Stents im expandierten Zustand;
- Fig. 2: eine Außenansicht des Stents von Fig. 1, wobei der Stent sich in einem überexpandierten Zustand befindet;
- Fig. 3: den Stent von Fig. 1 in einem komprimierten Zustand; und
- Fig. 4: einen Gefäßabzweigungs-Flächenbereich des Stents.

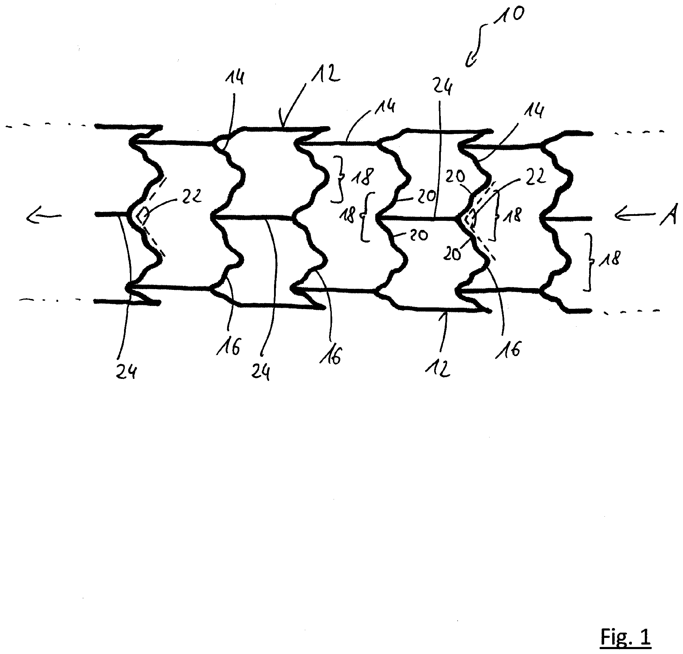

Die Fig. 1 zeigt einen einen Teilabschnitt eines Stents 10 in einem expandierten Zustand mit einem zweiten Querschnittsdurchmesser, welcher dem Nenndurchmesser des Stents 10 entspricht. Der Stent 10 umfasst einen röhrenförmigen Körper 12, der sich entlang einer axialen Richtung A erstreckt. Die Figuren zeigen den Stent 10 aus einer Blickrichtung senkrecht zur axialen Richtung A. Der Stent 10 wird durch eine Vielzahl von Umrandungselementen 14 gebildet, welche insgesamt eine gitterartige Struktur formen. Der Stent 10 erstreckt sich links und rechts des in Fig. 1 gezeigten Teilabschnitts fort.

Ein Teil der Umrandungselemente 14 bildet ringförmig um die axiale Richtung A umlaufende Stützstreben 16. Die Stützstreben 16 umfassen mehrere V-förmige Stützabschnitte 18, die derart hintereinander angeordnet sind, dass die Stützstreben 16 in Umfangsrichtung eine Zickzack-förmige Struktur aufweisen. Jeder V-förmige Stützabschnitt 18 umfasst zwei Schenkel 20, zwischen welchen ein Stegwinkel 22 definiert wird. Im gezeigten Ausführungsbeispiel beträgt der Stegwinkel 22 etwa zwischen 105° und 110°.

Im Bereich des Stegwinkels 22 sind die Stützstreben 16 mittels Längsverbindern 24 miteinander verbunden, wobei in Umfangsrichtung jeweils nur jeder zweite Stützabschnitt 18 mit einem Längsverbinder 24 gekoppelt ist. In Umfangsrichtung gesehen sind die Längsverbinder 24 jeweils abwechselnd auf gegenüberliegenden Seiten der Stützstrebe 16 angebracht.

Die Zickzack-Form der Stützstreben 16 ist derart gewählt, dass benachbarte Stützstreben 16 etwa parallel verlaufen, d.h. nicht zueinander versetzt bzw. verdreht sind. Dies bedeutet, dass die Verbindungsstellen jeweils zweier Schenkel 20 von verschiedenen Stützstreben 16 jeweils etwa entlang einer geraden Linie verlaufen, wobei entlang der geraden Linie die Stegwinkel 22 jeweils auf derselben Seite der Stützstreben 16 angeordnet sind.

Der gezeigte Stent 10 ist aus einem bioresorbierbaren Material gebildet, welches 97% Zink (Zn) und 3% Silber (Ag) aufweist. Durch die Wahl dieser Zink-Legierung lassen sich die gezeigten großen Stegwinkel 22 erreichen, ohne dass die Gefahr eines Bruchs der Umrandungselemente 14 besteht.

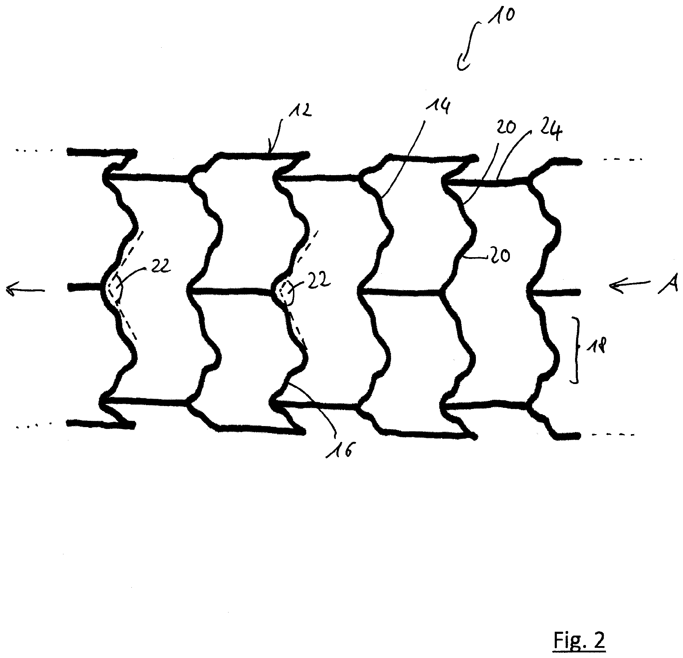

Wie in Fig. 2 gezeigt, ist es sogar möglich, den Stent zeitweilig in einen überexpandierten Zustand zu bringen, bei welchem der Querschnittsdurchmesser zumindest 7 bis 20% größer als der vorgenannte zweite Querschnittsdurchmesser ist. Ein solcher Zustand ist in Fig. 2 gezeigt. Wie in Fig. 2 dargestellt, kann in diesem Zustand der Stegwinkel 22 mehr als 120° betragen.

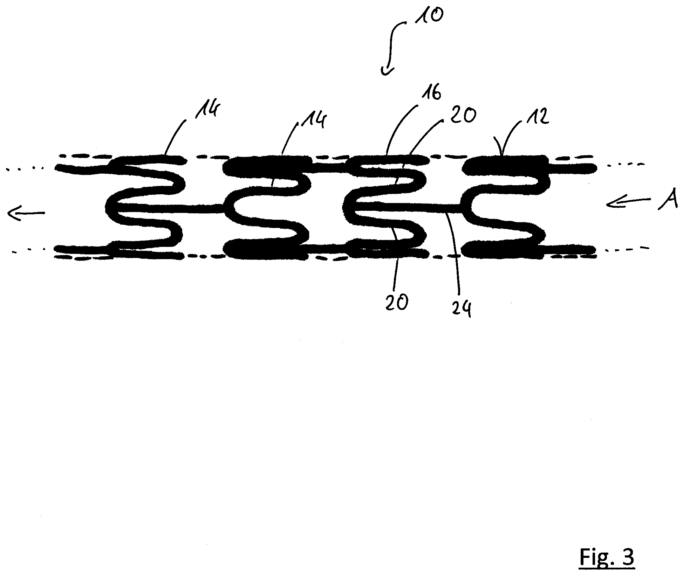

Demgegenüber zeigt Fig. 3 den Stent 10 im komprimierten Zustand mit einem ersten Querschnittsdurchmesser. In diesem Zustand sind die Schenkel 20 annähernd parallel, so dass der Stegwinkel 20 nicht existiert bzw. annähernd 0° ist.

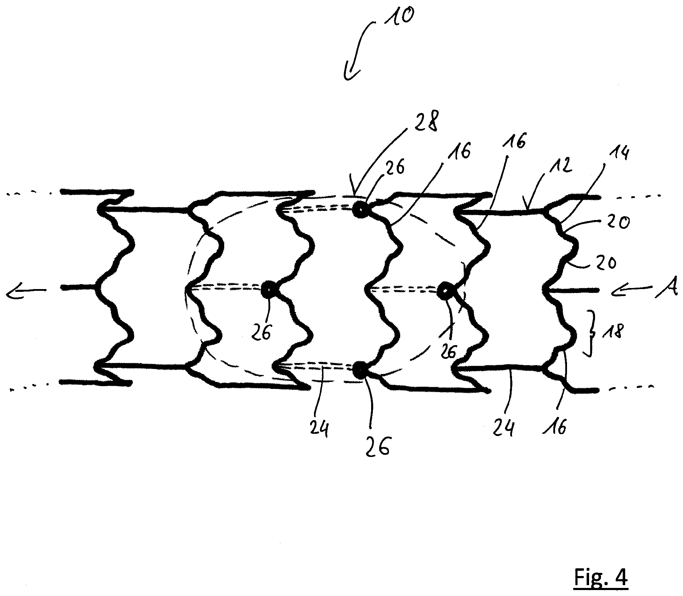

Fig. 4 zeigt schließlich einen anderen Teilabschnitt des Stents 10, als in den Fig. 1-3 gezeigt. Fig. 4 zeigt einen Teilabschnitt des Stents 10 mit einem in Draufsicht etwa kreisförmigen Gefäßabzweigungs-Flächenbereich 28.

Die Stützstreben 16 sind innerhalb des Gefäßabzweigungs-Flächenbereichs 28 um 25% dünner ausgeführt als beim übrigen Stent 10. Zudem sind innerhalb des Gefäßabzweigungs-Flächenbereichs 28 keine Längsverbinder 24 angeordnet, was durch gestrichelt gezeichnete (nicht existente) Längsverbinder 24 angedeutet wird. Es versteht sich, dass die Stützstreben 16, welche durch den Gefäßabzweigungs-Flächenbereichs 28 verlaufen, durch außerhalb des Gefäßabzweigungs-Flächenbereichs 28 angeordnete Längsverbinder 24 gekoppelt sind. Diese sind in Fig. 4 nicht gezeigt. Der Verzicht auf die Längsverbinder 24 und die dünnere Ausführung der Stützstreben 16 führt zu einer leichteren Dilatierbarkeit des Stents im Bereich des Gefäßabzweigungs-Flächenbereichs 28, so dass ich die Stützstreben 16 im Bereich einer Gefäßabzweigung nach außen gebogen werden können, d.h. an den Rand des Gefäßabzweigungs-Flächenbereichs 28 gebogen werden können, um beispielsweise einen Blutfluss durch die Gefäßabzweigung zu verbessern.

Im Bereich der entfallenen Längsverbinder 24 sind insgesamt vier kreisförmige oder elliptische Positionsmarker 26 an den Stützstreben 16 angebracht. Die Positionsmarker erleichtern das Positionieren des Stents 10 auf einem Katheter (nicht gezeigt).

### Bezugszeichenliste

- 10: Stent
- 12: röhrenförmiger Körper
- 14: Umrandungselement
- 16: Stützstrebe
- 18: V-förmiger Stützabschnitt
- 20: Schenkel
- 22: Stegwinkel
- 24: Längsverbinder
- 26: Positionsmarker
- 28: Gefäßabzweigungs-Flächenbereich
- A: axiale Richtung

## Patentansprüche

1. Stent (10) zur transluminalen Implantation in Hohlorgane, insbesondere in Blutgefäße, Harnleiter, Speiseröhren, Colon, Duodenum oder Gallenwege, mit einem im Wesentlichen röhrenförmigen Körper, der sich entlang einer axialen Richtung (A) erstreckt und von einem komprimierten Zustand mit einem ersten Querschnittsdurchmesser in einen expandierten Zustand mit einem vergrößerten zweiten Querschnittsdurchmesser überführbar ist, wobei der Stent (10) eine Vielzahl von Zellen umfasst, welche von durch den röhrenförmigen Körper gebildeten stegartigen Umrandungselementen (14) definiert werden,
wobei die Umrandungselemente (14) zumindest eine ringförmig um die axiale Richtung (A) umlaufende Stützstrebe (16) umfassen, und
wobei die Stützstrebe (16) zumindest einen V-förmigen Stützabschnitt (18) aufweist, welcher einen Stegwinkel (22) von 90° bis 150° umfasst, wenn der Stent (10) den zweiten Querschnittsdurchmesser aufweist,
**dadurch gekennzeichnet, dass**
im Bereich des Stegwinkels (22) ein Längsverbinder (24) an der Stützstrebe (16) angebracht ist, welcher die Stützstrebe (16) mit zumindest einer weiteren Stützstrebe (16) verbindet und die Umrandungselemente (14) zumindest bereichsweise ein bioresorbierbares Material umfassen, welches Zink umfasst.

2. Stent (10) nach Anspruch 1,
**dadurch gekennzeichnet , dass**
der Stegwinkel (22) einen Winkel von 100° bis 130°, bevorzugt von 105° bis 115°, aufweist.

3. Stent (10) nach Anspruch 1 oder 2,
**dadurch gekennzeichnet , dass**
das bioresorbierbare Material aus Zink und Silber besteht, wobei das bioresorbierbare Material 90,0 bis 99,95 Massen-% Zink und 0,05 bis 10,0 Massen-% Silber enthält.

4. Stent (10) nach zumindest einem der vorstehenden Ansprüche,
**dadurch gekennzeichnet , dass**
die Stützstrebe (16) zumindest im Wesentlichen derart radial um die axiale Richtung (A) umläuft, dass die axiale Richtung einen Normalenvektor auf die von der Stützstrebe (16) definierte Ebene bildet.

5. Stent (10) nach zumindest einem der vorstehenden Ansprüche,
**dadurch gekennzeichnet , dass**
der Stent (10) auf einen dritten Querschnittsdurchmesser aufweitbar ist, welcher größer als der zweite Querschnittsdurchmesser ist, wobei der Stegwinkel (22) sich auf zumindest 120°, bevorzugt auf zumindest 140° oder 160°, vergrößert, wenn der Stent (10) den dritten Querschnittsdurchmesser aufweist.

6. Stent (10) nach zumindest einem der vorstehenden Ansprüche,
**dadurch gekennzeichnet , dass**
innerhalb eines vordefinierten Gefäßabzweigungs-Flächenbereichs (28) die Umrandungselemente (14) eine geringere Masse aufweisen, als die Umrandungselemente (14) in einem anderen Flächenbereich gleicher Größe, wobei die geringere Masse beispielsweise durch dünnere Umrandungselemente (14) erzielt wird.

7. Stent (10) nach Anspruch 6,
**dadurch gekennzeichnet , dass**
innerhalb des Gefäßabzweigungs-Flächenbereichs (28) keine Längsverbinder (24) angeordnet sind.

8. Stent (10) nach Anspruch 6 oder 7,
**dadurch gekennzeichnet , dass**
innerhalb des Gefäßabzweigungs-Flächenbereichs (28) und/oder angrenzend an den Gefäßabzweigungs-Flächenbereich ein oder mehrere Röntgenmarker angeordnet sind.

9. Stent (10) nach zumindest einem der vorstehenden Ansprüche,
**dadurch gekennzeichnet , dass**
die Umrandungselemente (14) zumindest bereichsweise mit einem Medikament versehen sind.

10. Stent (10) nach zumindest einem der vorstehenden Ansprüche,
**dadurch gekennzeichnet , dass**
die Umrandungselemente (14) ein Wandstärke von maximal 4%, bevorzugt von maximal 2%, weiter bevorzugt von maximal 1,5%, des zweiten Querschnittsdurchmessers aufweisen.

11. Stent (10) zur transluminalen Implantation in Hohlorgane, insbesondere in Blutgefäße, Harnleiter, Speiseröhren, Colon, Duodenum oder Gallenwege, mit einem im Wesentlichen röhrenförmigen Körper, der sich entlang einer axialen Richtung (A) erstreckt und von einem komprimierten Zustand mit einem ersten Querschnittsdurchmesser in einen expandierten Zustand mit einem vergrößerten zweiten Querschnittsdurchmesser überführbar ist, wobei der Stent (10) eine Vielzahl von Zellen umfasst, welche von durch den röhrenförmigen Körper gebildeten stegartigen Umrandungselementen (14) definiert werden,
wobei die Umrandungselemente (14) zumindest eine ringförmig um die axiale Richtung (A) umlaufende Stützstrebe (16) umfassen, und
wobei ein Längsverbinder (24) an der Stützstrebe (16) angebracht ist, welcher die Stützstrebe (16) mit zumindest einer weiteren Stützstrebe (16) verbindet,
**dadurch gekennzeichnet, dass**
innerhalb eines vordefinierten Gefäßabzweigungs-Flächenbereichs (28) keine Längsverbinder (24) angeordnet sind und
die Umrandungselemente (14) zumindest bereichsweise ein bioresorbierbares Material umfassen, welches Zink umfasst.

12. Stent (10) nach Anspruch 11,
**dadurch gekennzeichnet , dass**
innerhalb des Gefäßabzweigungs-Flächenbereichs (28) und/oder angrenzend an den Gefäßabzweigungs-Flächenbereich ein oder mehrere Röntgenmarker oder Positionsmarker (26) angeordnet sind.

13. Stent (10) nach Anspruch 11 oder 12,
**dadurch gekennzeichnet , dass**
innerhalb des vordefinierten Gefäßabzweigungs-Flächenbereichs (28) die Umrandungselemente (14) eine geringere Masse aufweisen, als die Umrandungselemente (14) in einem anderen Flächenbereich gleicher Größe, wobei die geringere Masse beispielsweise durch dünnere Umrandungselemente (14) erzielt wird.

## Claims

1. A stent (10) for transluminal implantation into hollow organs, in particular into blood vessels, ureters, esophagi, the colon, the duodenum or the biliary tract, comprising a substantially tubular body which extends along an axial direction (A) and which can be converted from a compressed state having a first cross-sectional diameter into an expanded state having an enlarged second cross-sectional diameter,
wherein the stent (10) comprises a plurality of cells which are defined by strut-like bordering elements (14) formed by the tubular body,
wherein the bordering elements (14) comprise at least one support strut (16) revolving in a ring shape about the axial direction (A), and
wherein the support strut (16) has at least one V-shaped support section (18) which comprises a strut angle (22) of 90° to 150° when the stent (10) has the second cross-sectional diameter,
**characterized in that**
a longitudinal connector (24) is attached to the support strut (16) in the region of the strut angle (22) and connects the support strut (16) to at least one further support strut (16) and the bordering elements (14) at least regionally comprise a bioresorbable material which comprises zinc.

2. A stent (10) in accordance with claim 1,
**characterized in that**
the strut angle (22) has an angle of 100° to 130°, preferably of 105° to 115°.

3. A stent (10) in accordance with claim 1 or claim 2,
**characterized in that**
the bioresorbable material consists of zinc and silver, wherein the bioresorbable material includes 90.0 to 99.95 mass % zinc and 0.05 to 10.0 mass % silver.

4. A stent (10) in accordance with at least one of the preceding claims,
**characterized in that**
the support strut (16) at least substantially revolves radially about the axial direction (A) such that the axial direction forms a normal vector to the plane defined by the support strut (16).

5. A stent (10) in accordance with at least one of the preceding claims,
**characterized in that**
the stent (10) is expandable to a third cross-sectional diameter which is larger than the second cross-sectional diameter, with the strut angle (22) increasing to at least 120°, preferably to at least 140° or 160°, when the stent (10) has the third cross-sectional diameter.

6. A stent (10) in accordance with at least one of the preceding claims,
**characterized in that**
the bordering elements (14) have a smaller mass within a predefined vessel branching areal region (28) than the bordering elements (14) in another areal region of the same size, with the smaller mass, for example, being achieved by thinner bordering elements (14).

7. A stent (10) in accordance with claim 6,
**characterized in that**
no longitudinal connectors (24) are arranged within the vessel branching areal region (28).

8. A stent (10) in accordance with claim 6 or claim 7,
**characterized in that**
one or more X-ray markers are arranged within the vessel branching areal region (28) and/or adjacent to the vessel branching areal region.

9. A stent (10) in accordance with at least one of the preceding claims,
**characterized in that**
the bordering elements (14) are at least regionally provided with a medicine.

10. A stent (10) in accordance with at least one of the preceding claims,
**characterized in that**
the bordering elements (14) have a wall thickness of at most 4%, preferably of at most 2%, further preferably of at most 1.5%, of the second cross-sectional diameter.

11. A stent (10) for transluminal implantation into hollow organs, in particular into blood vessels, ureters, esophagi, the colon, the duodenum or the biliary tract, comprising a substantially tubular body which extends along an axial direction (A) and which can be converted from a compressed state having a first cross-sectional diameter into an expanded state having an enlarged second cross-sectional diameter,
wherein the stent (10) comprises a plurality of cells which are defined by strut-like bordering elements (14) formed by the tubular body,
wherein the bordering elements (14) comprise at least one support strut (16) revolving in a ring shape about the axial direction (A), and
wherein a longitudinal connector (24) is attached to the support strut (16) and connects the support strut (16) to at least one further support strut (16), **characterized in that**
no longitudinal connectors (24) are arranged within a predefined vessel branching areal region (28), and
the bordering elements (14) at least regionally comprise a bioresorbable material which comprises zinc.

12. A stent (10) in accordance with claim 11,
**characterized in that**
one or more X-ray markers or position markers (26) are arranged within the vessel branching areal region (28) and/or adjacent to the vessel branching areal region.

13. A stent (10) in accordance with claim 11 or claim 12,
**characterized in that**
the bordering elements (14) have a smaller mass within the predefined vessel branching areal region (28) than the bordering elements (14) in another areal region of the same size, with the smaller mass, for example, being achieved by thinner bordering elements (14).

## Revendications

1. Stent (10) pour implantation transluminale dans des organes creux, en particulier dans les vaisseaux sanguins, les uretères, l'œsophage, le côlon, le duodénum ou les voies biliaires, comprenant un corps sensiblement tubulaire qui s'étend le long d'une direction axiale (A) et peut être transféré d'un état comprimé, présentant un premier diamètre de section transversale, à un état déployé, présentant un deuxième diamètre de section transversale plus important, le stent (10) comprenant une multitude de cellules définies par des éléments de bordure (14) en forme d'âme formés par le corps tubulaire,
dans lequel
les éléments de bordure (14) comprennent au moins une entretoise de soutien (16) s'étendant en forme d'anneau autour de la direction axiale (A), et l'entretoise de soutien (16) comprend au moins une portion de soutien en forme de V (18) qui présente une équerre d'âme (22) de 90° à 150° lorsque le stent (10) présente le deuxième diamètre de section transversale, **caractérisé en ce que**
dans la zone de l'équerre d'âme (22), un connecteur longitudinal (24) est fixé à l'entretoise de soutien (16), lequel relie l'entretoise de soutien (16) à au moins une autre entretoise de soutien (16), et les éléments de bordure (14) comprennent au moins localement un matériau biorésorbable qui contient du zinc.

2. Stent (10) selon la revendication 1,
**caractérisé en ce que**
l'équerre d'âme (22) présente un angle de 100° à 130°, de préférence de 105° à 115°.

3. Stent (10) selon la revendication 1 ou 2,
**caractérisé en ce que**
le matériau biorésorbable est constitué de zinc et d'argent, le matériau biorésorbable contenant de 90,0 à 99,95 % en masse de zinc et de 0,05 à 10,0 % en masse d'argent.

4. Stent (10) selon l'une au moins des revendications précédentes,
**caractérisé en ce que**
l'entretoise de soutien (16) s'étend au moins sensiblement radialement autour de la direction axiale (A) de telle sorte que la direction axiale forme un vecteur normal au plan défini par l'entretoise de soutien (16).

5. Stent (10) selon l'une au moins des revendications précédentes,
**caractérisé en ce que**
le stent (10) peut être dilaté jusqu'à un troisième diamètre de section transversale qui est supérieur au deuxième diamètre de section transversale, l'équerre d'âme (22) augmentant alors à au moins 120°, de préférence à au moins 140° ou 160°, lorsque le stent (10) présente le troisième diamètre de section transversale.

6. Stent (10) selon l'une au moins des revendications précédentes,
**caractérisé en ce que**
à l'intérieur d'une zone surfacique prédéfinie de ramification vasculaire (28), les éléments de bordure (14) présentent une masse inférieure à celle des éléments de bordure (14) situés dans une autre zone surfacique de même taille, la masse inférieure étant obtenue, par exemple, par des éléments de bordure (14) plus minces.

7. Stent (10) selon la revendication 6,
**caractérisé en ce que**
aucun connecteur longitudinal (24) n'est disposé à l'intérieur de la zone surfacique de ramification vasculaire (28).

8. Stent (10) selon la revendication 6 ou 7,
**caractérisé en ce que**
un ou plusieurs marqueurs radiographiques sont disposés à l'intérieur de la zone surfacique de ramification vasculaire (28) et/ou à proximité de la zone surfacique de ramification vasculaire.

9. Stent (10) selon l'une au moins des revendications précédentes,
**caractérisé en ce que**
les éléments de bordure (14) sont pourvus au moins localement d'un médicament.

10. Stent (10) selon l'une au moins des revendications précédentes,
**caractérisé en ce que**
les éléments de bordure (14) présentent une épaisseur de paroi de 4 % au maximum, de préférence de 2 % au maximum, de manière particulièrement préférée de 1,5 % au maximum, du deuxième diamètre de section transversale.

11. Stent (10) pour implantation transluminale dans des organes creux, en particulier dans les vaisseaux sanguins, les uretères, l'œsophage, le côlon, le duodénum ou les voies biliaires, comprenant un corps sensiblement tubulaire qui s'étend le long d'une direction axiale (A) et peut être transféré d'un état comprimé, présentant un premier diamètre de section transversale, à un état déployé, présentant un deuxième diamètre de section transversale plus important, le stent (10) comprenant une multitude de cellules définies par des éléments de bordure (14) en forme d'âme formés par le corps tubulaire,
dans lequel
les éléments de bordure (14) comprennent au moins une entretoise de soutien (16) s'étendant en forme d'anneau autour de la direction axiale (A), et un connecteur longitudinal (24) est fixé à l'entretoise de soutien (16), lequel relie l'entretoise de soutien (16) à au moins une autre entretoise de soutien (16),
**caractérisé en ce que**
aucun connecteur longitudinal (24) n'est disposé à l'intérieur d'une zone surfacique prédéfinie de ramification vasculaire (28), et
les éléments de bordure (14) comprennent au moins localement un matériau biorésorbable qui contient du zinc.

12. Stent (10) selon la revendication 11,
**caractérisé en ce que**
un ou plusieurs marqueurs radiographiques ou marqueurs de position (26) sont disposés à l'intérieur de la zone surfacique de ramification vasculaire (28) et/ou à proximité de la zone surfacique de ramification vasculaire.

13. Stent (10) selon la revendication 11 ou 12,
**caractérisé en ce que**
à l'intérieur de la zone surfacique prédéfinie de ramification vasculaire (28), les éléments de bordure (14) ont une masse inférieure à celle des éléments de bordure (14) situés dans une autre zone surfacique de même taille, la masse inférieure étant obtenue, par exemple, par des éléments de bordure (14) plus minces.
